# NEUE EUROPÄISCHE PATENTSCHRIFT

(11) **EP 1 814 609 B2**
(45) Veröffentlichungstag und Bekanntmachung des Hinweises auf die Entscheidung über den Einspruch: **21.09.2011**
(45) Hinweis auf die Patenterteilung: 02.04.2008
(21) Anmeldenummer: 05806372.8
(22) Anmeldetag: 02.11.2005
(51) Int. Cl.: A61M 1/00, A61M 27/00

(54) **DRAINAGEVORRICHTUNG ZUR WUNDBEHANDLUNG UNTER EINSATZ VON UNTERDRUCK**
DRAINAGE DEVICE FOR THE TREATMENT OF WOUNDS WITH APPLICATION OF A VACUUM
DISPOSITIF DE DRAINAGE SERVANT A SOIGNER DES PLAIES PAR L'APPLICATION D'UNE PRESSION NEGATIVE

(30) Priorität: 24.11.2004 DE 202004018245 U
(43) Veröffentlichungstag der Anmeldung: 08.08.2007
(73) Patentinhaber: Riesinger, Birgit, 48348 Ostbevern (DE)
(72) Erfinder: Riesinger, Birgit, 48348 Ostbevern (DE)
(74) Vertreter: Michalski Hüttermann & Partner Patentanwälte
(86) Internationale Anmeldenummer: PCT/EP2005/011702
(87) Internationale Veröffentlichungsnummer: WO 2006/056294

(56) Entgegenhaltungen:
- EP-A2- 1 129 734
- WO-A1-01/89431
- WO-A1-96/05873
- WO-A1-99/01173
- WO-A1-03/094813
- WO-A1-2005/123170
- AT-T- 33 446
- DE-A1- 10 059 439
- DE-A1- 19 517 699
- DE-C2- 2 953 373
- DE-T2- 3 850 798
- US-A- 5 086 763
- US-A- 5 549 584
- US-A1- 2004 054 338

## Beschreibung

Die Erfindung betrifft eine Drainagevorrichtung zur Wundbehandlung unter Einsatz von Unterdruck, aufweisend:
- ein gasdichtes, aus folienartigem Material bestehendes Wundabdeckungselement, das im am Körper des Patienten angelegten Zustand an der Hautoberfläche um den Wundbereich herum adhäsiv befestigt ist und einen zwischen der jeweiligen Wunde und dem Wundabdeckungselement verbleibenden, abgedichteten Wundraum bildet,
- wenigstens einen an ein den Unterdruck erzeugendes Mittel, wie Vakuumpumpe, anschließbaren Drainageschlauch, der in den Wundraum etwa parallel zum Wundabdeckungselement liegend einsetzbar ist, über den die im Wundraum befindlichen Stoffe evakuiert werden können,
- und wenigstens einen innerhalb des Wundraumes angeordneten, die Wundsekrete aufsaugenden Absorptionskörper.

Eine Vorrichtung der eingangs genannten Art ist aus der DE 195 17 699 bekannt. Die bekannte Vorrichtung weist eine Abdeckfolie zum flächigen Überdecken und luftdichten Abschließen der Wunde auf, so dass unterhalb der Abdeckfolie im Bereich der Wunde ein Wundraum entsteht, in den eine Schaumstoff-Einlage und ein in diese eingezogener Drainageschlauch eingesetzt sind. Mit Hilfe der Schaumstoff-Einlage sollen die Wundsekrete gleichmäßiger über die gesamte Wundoberfläche abgesaugt werden. An den Drainageschlauch ist wiederum ein Auffangbehälter mit Vakuumpumpe zur Erzeugung des Unterdrucks angeschlossen. Wenn der Auffangbehälter mit Wundsekret gefüllt ist, wird die gesamte Vorrichtung entsorgt. Der Zweck der Einmalgerätes ist, die Wundsekrete kontinuierlich durch den Drainageschlauch dem Auffangbehälter zuzuleiten.

Die US 55 49 584 zeigt eine Vorrichtung zur Vakuumtherapie, welche aus einer Wundabdeckung, einer am Saugrohr angeordneten Membranpumpe und einem der Membranpumpe nachgeschalteten, beutelartigen Kollektor besteht. Unterhalb der Wundabdeckung ist eine Materialschicht oder eine lose Schüttung von flüssigkeitsabsorbierenden Fasern angeordnet, die auf einer perforierten Schicht aufliegt, welche wiederum von einer weiteren, adhäsiven Schicht unterlegt ist. Die Fasern sind noch durch eine flüssigkeitspermeable, mehrere Fenster aufweisende, obere Schicht bedeckt, wobei die Fenster jeweils von einem ebenso flüssigkeitsdurchlässigen Materialabschnitt unterlegt sind. Das Saugrohr ist mit einem oberhalb der Wundabdeckung angeordneten Verbindungsstück verbunden und hat keinen direkten Kontakt mit dem sich innerhalb des Wundraumes befindenden Absorptionsmaterial. Die so konstruierte Vorrichtung scheint kompliziert und teuer in Herstellung zu sein.

In DE 29 53 373 C2 ist ebenfalls eine Vorrichtung zur Wundbehandlung unter Einsatz von Unterdruck gezeigt, bestehend aus einem Wundabdeckungselement, einer darunter liegenden Schaumstoff-Einlage und wenigstens einer mit den Poren der Schaumstoff-Einlage kommunizierten Schlauchleitung.

In DE 38 50 798 T2 ist eine sterile, quaderförmige, jedoch starre Kassette mit einem darin untergebrachten chirurgischen Verband dargestellt. Die Kassette wird mit ihrem unteren Rand auf der Haut des Patienten befestigt. Die Kassette ist zum Umschließen des Operationsfeldes am Patienten oben und unten offen.

In US 50 86 763 A ist eine an der Haut des Patienten befestigbare, ebenfalls kassettenartige Wundbehandlungsvorrichtung gezeigt. Ein auswechselbarer Wundverband ist auf der Unterseite eines Schwenkdeckels zu platzieren.

Eine aus AT E33 446 B bekannte, beutelartige Wundbehandlung- und Wunddrainagevorrichtung weist einen unteren, umlaufenden Rand zum Ankleben an die Haut des Patienten, einen Katheterhalter, zwei an einer oberen Wand angeordnete Behandlungsöffnungen mit Kappen sowie eine an der unteren Wand des Beutels liegende, den Zugang zu der Wunde ermöglichende Öffnung auf. Die Schrift beschreibt lediglich die Konstruktion des Beutels.

Aufgabe der Erfindung ist, eine kostengünstigere Vorrichtung zur Vakuumtherapie von Wunden zu konzipieren, deren Aufbau vereinfacht ist.

Diese Aufgabe ist durch eine Vorrichtung mit den Merkmalen des Anspruchs 1 gelöst.

Das Wundabdeckungselement kann über eine biegsame Anschlussplatte, sogenannte Basisplatte, am Körper des Patienten befestigbar sein, an der eine oder mehrere, an die Wundfläche (-en) angepassten Öffnungen vor dem Aufkleben an die Haut ausgeschnitten werden. Es ist jedoch nicht ausgeschlossen, auf die Basisplatte zu verzichten, wenn am Rand des Wundabdeckungselementes eine umlaufende Klebefläche eingebracht wird, vorausgesetzt, dass die Klebesubstanz gesundheitsunbedenklich und am Wundabdeckungselement eine entsprechende Anschlussstelle zum Einführen des Drainageschlauchs vorgesehen ist. Bevorzugt wird jedoch ein marktüblicher Versorgungsbeutel mit gasdichtem Behandlungsfenster und Abfluss, dessen dem Wundabdeckungselement gegenüber liegende Seitenwand mit der Anschlussplatte werkseitig verklebt ist.

Wichtig ist, dass innerhalb des Versorgungsbeutels wenigstens ein mit Superabsorbentien angereicherter, auswechselbarer Absorptionskörper und ein Drainageschlauch angeordnet ist, welcher Drainageschlauch auf dem Absorptionskörper liegt.

Der Drainageschlauch kann aber auch zwischen zwei eine Sandwichanordnung bildenden Absorptionskörpern liegen, die gleiche oder unterschiedliche Saugkraft aufweisen. Bei der Sandwichanordnung kann ein mit Superabsorbentien angereicherten Absorptionskörper und ein zusätzlicher, nicht mit Superabsorbentien versehener Absorptionskörper, beispielsweise ein flacher, offenporiger Schaumstoffkörper zum Einsatz kommen. Hierbei sind unterschiedliche Möglichkeiten vorgesehen, nämlich eine Sandwichanordnung, bei der der Schaumstoffkörper zwischen der Anschlußplatte und dem mit Superabsorbentien angereicherten Absorptionskörper oder zwischen dem Wundabdeckungselement und dem mit Superabsorbentien angereicherten Absorptionskörper liegt.

Durch den Auswahl von Absorptionskörpern und ihrer Saugkraft kann ein optimaler Wundheilungsprozess "programmiert" werden.

Um die Keime abzutöten, kann der umhüllte Absorptionskörper und/oder der zusätzliche, schaumstoffartige Absorptionskörper und/oder das antiadhäsive Folienelement mit silber-, kupfer- oder zinkhaltigen Substanzen versehen sein, beispielsweise in nanokristalliner Form.

Wichtig ist auch, dass die Fläche des mit Superabsorberteilchen angereicherten Textilabschnittes des Absorptionskörpers wesentlich kleiner als die der Hülle ist, damit sich der Absorptionskörper in der Nähe seiner gesamten Füllungskapazität in seinem Querschnitt ungehindert einer Kreisform annähern kann. Die Ausschöpfung der maximalen oder nahezu maximalen Füllungskapazität des Absorptionskörpers trägt der Kostensenkung der Wundbehandlung bei.

Das Wundabdeckungselement kann an wenigstens einem Teil seiner Fläche transparent sein, damit der Zustand des Wundheilungsprozesses beobachtet werden kann.

Die Drainagevorrichtung kann mit einem flüssigkeitsdurchlässigen, schleimhautfreundlichen Schutzelement versehen sein, das auf einer dem Wundabdeckungselement gegenüber liegenden Seite des Absorptionskörpers angeordnet ist und das flächenmäßig etwa dem umhüllten Absorptionskörper gleich ist. Das Schutzelement kann auch aus weichem, offenzelligen Schaumstoff oder einem sehr lockeren Vliesgebilde hergestellt sein. Schließlich kann das Schutzelement eine unterhalb des Absorptionskörpers liegende, lose Schüttung aus Vlies- oder Schaumstoffstücken sein, die nach dem beendeten Absorptionsvorgang aus der Wunde beispielsweise mit einer Pinzette entfernt werden können. Darüber hinaus erfüllt ein voluminöses Gebilde nicht nur die die Schleimhaut schützende Funktion, sondern auch die Funktion eines Absorbers. Der offenzellige Schaumstoff oder das Vliesgebilde können mehrfach größere Poren als die der Hülle haben, so dass das die größeren Partikeln von Wundexsudat aufgenommen werden können.

Der Unterdruck im Wundraum unterhalb des Wundabdeckungselementes kann - bei am Körper des Patienten angeklebter Vorrichtung - manuell oder mechanisch bzw. elektrisch erzeugt werden. Eine einfachste manuelle Vakuumerzeugung kann beispielsweise mithilfe einer sogenannten Scheerengriff-Vakuumpumpe oder eines bekannten, per Hand zusammendrückbaren Gummibalgs ("Ballpumpe") erfolgen. Zur elektrischen Vakuumerzeugung eignen sich unterschiedliche, marktübliche Vakuumpumpen, die z. B. mit Schlauch und Druckregulierer lieferbar sind.

Bei einer stationären, z. B. postoperativen Wundbehandlung kann der Unterdruck durch Anschließen der Drainagevorrichtung an eine vorhandene, stationäre klinische Vakuuminstallation, gegebenenfalls über einen Druckregler erzeugt werden.

Die Vorrichtung kann in Draufsicht auf ihre Flachseite polygonal, oval oder kreisförmig sein. Sie kann auch in Draufsicht auf ihre Flachseite den bekannten Versorgungsbeuteln ähnlich, d. h. "flaschenförmig" sein.

Die Hülle des Absorptionskörpers besteht aus einem flüssigkeitsdurchlässigen, schleimhautverträglichen Natur- oder Kunststoff, an dem die Wundsekrete nicht oder kaum haften. Dies ermöglicht den Transport von flüssigen Wundsekreten in das Absorptionsmaterial. Die Wundsekrete treten durch die Hülle hindurch und werden durch das mit Superabsorbentien angereichertes Absorptionsmaterial aufgesaugt.

Vorteilhaft ist, an der Hülle des Absorptionskörpers einen peripheren Überstand an Hüllenmaterial vorzusehen, damit eine schmerzhafte Berührung der relativ harten Naht mit der Wundoberfläche begrenzt oder gar vermieden werden kann. Hierbei wird als Überstand das Hüllenmaterial zwischen einer umlaufenden Naht und dem äußersten Umfang der Hülle verstanden.

Die Hülle des Absorptionskörpers kann mit einem Zugmittel versehen sein, damit der verbrauchte, aufgequollene Absorptionskörper einfacher aus dem Wundbereich herausgezogen werden kann.

Sowohl die Hülle, als auch das innerhalb der Hülle liegende Absorptionsmaterial kann mit einem geruchshemmenden und/oder -neutralisierenden bzw. -maskierenden Zusatz versehen sein, beispielweise mit Aktivkohlefilter.
Von großem Vorteil ist, dass bei Gasevakuierung keine Wundsekretpartikeln mitgerissen werden. Diese verbleiben solange innerhalb der Hülle des Absorptionskörpers bis die ganze Vorrichtung aus dem Körper des Patienten entfernt und entsorgt bzw. der aufgequollene Absorptionskörper ausgewechselt wird.

Insgesamt ist eine Vorrichtung zur Wundbehandlung unter Einsatz von Unterdruck geschaffen, bei der die Wundsekrete durch den mit Superabsorber durchsetze Vlies des Absorptionskörpers angesaugt werden und innerhalb der den Absorptionskörper umgebenden Hülle verbleiben, ohne aus der Hülle in den abgedeckten Wundraum zurück zu gelangen,
wobei mit zunehmender Absorption die Querschnittsfläche des Absorptionskörpers sich stark, mehrfach vergrößert und einer Kreisform annähert, und wobei beim Aufrechterhalten des Unterdrucks mithilfe einer an die Vorrichtung anschließbaren Vakuumpumpe, d. h. bei Gasevakuierung im wesentlichen keine Wundsekrete mitgerissen werden. Darüber hinaus ist es möglich, auf einen zusätzlichen Auffangbehälter zu verzichten.

Soll ein Überschuss an Wundsekret abgeführt werden, so empfiehlt sich, den Drainageschlauch über ein Rückschlagventil an einen Auffangbehälter anzuschließen, der wiederum einer Vakuumpumpe mit Druckmanometer vorgeschaltet ist. In diesem Fall dient der auswechselbare Absorptionskörper als Zwischenspeicher für die aus der Wunde austretenden Wundsekrete.

Die Drainagevorrichtung gemäß der Erfindung kann auch mit der Kompressions-Therapie verwendet werden, beispielsweise bei der Behandlung von offenen Beinen.

Ein Ausführungsbeispiel der Erfindung ist anhand der Zeichnung näher erläutert. Die Figuren zeigen:
- Fig. 1: eine Drainagevorrichtung in Draufsicht auf das Wundabdeckungselement;
- Figuren 2a bis 2d: die an die Haut des Patienten angeklebte Vorrichtung gemäß der Fig. 1, in einem schematischen Schnitt;
- Fig. 3: die Vorrichtung gemäß der Fig. 1, jedoch mit einem Hals, ebenso in Draufsicht auf ihre Flachseite;
- Figuren 4a bis 4c: einen Schnitt A-A gemäß der Fig. 3 mit drei unterschiedlichen Sandwichanordnungen zweier Absorptionskörper;
- Fig. 5: die Vorrichtung gemäß der Fig. 1 oder 3 in einem schematischen Längsschnitt, angeschlossen an eine tragbare oder eine stationäre Vakuuminstallation;
- Fig. 6a: eine weitere Ausführungsform der Drainagevorrichtung, in Draufsicht auf das Wundabdeckungselement;
- Fig. 6b: einen Schnitt gemäß der Fig. 6a.

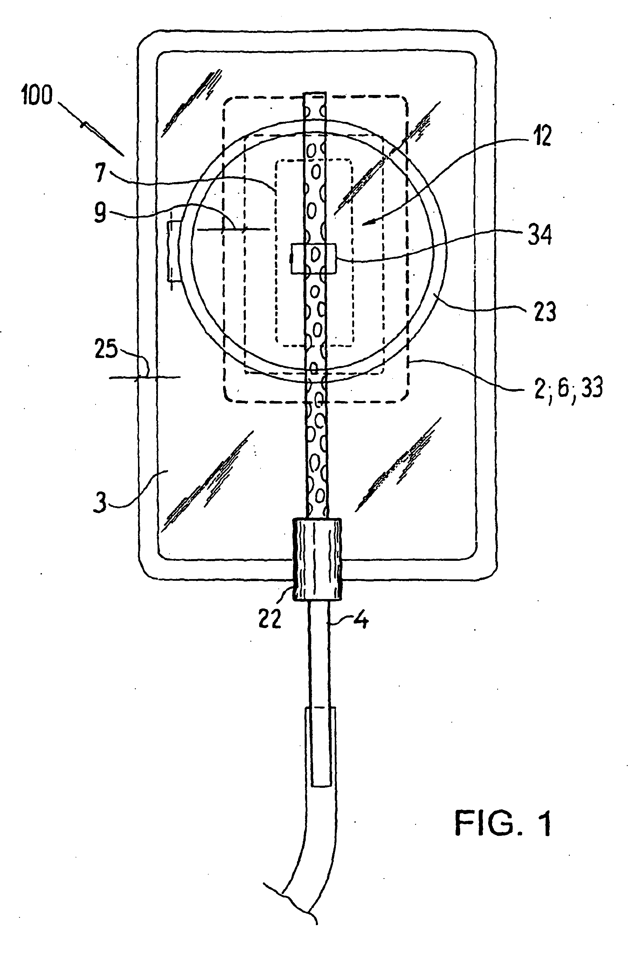

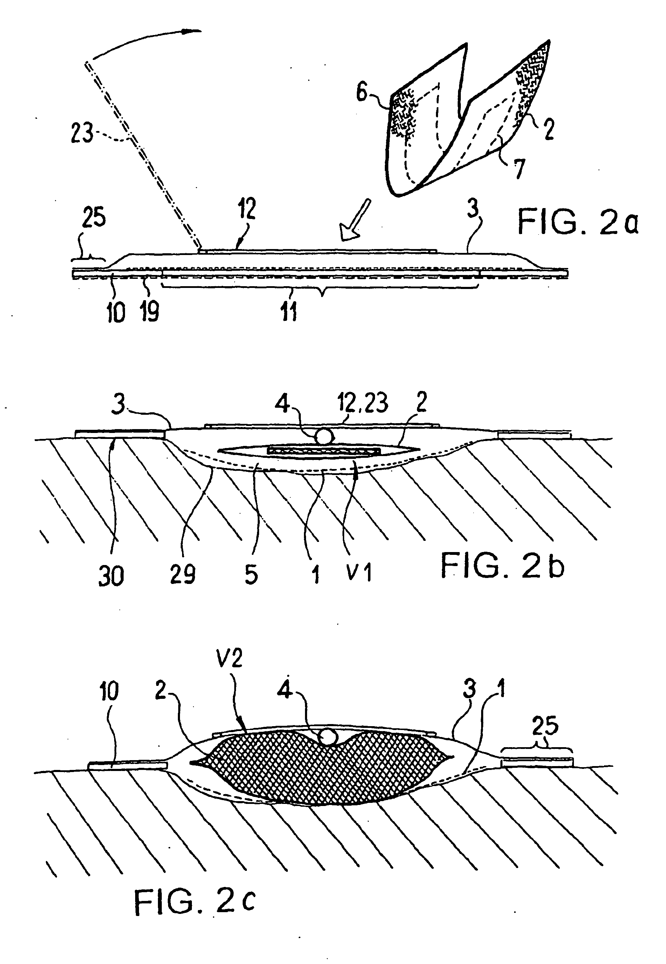

In den Figuren 1 und 2a bis 2d ist eine Drainagevorrichtung 100 zur Wundbehandlung unter Einsatz von Unterdruck dargestellt, bestehend aus einem rechteckigen, folienartigen, transparenten Wundabdeckungselement 3, einer aus hydrokolloidartigen Anschlussplatte 10, einem dazwischen liegenden Absorptionskörper 2 und einem Drainageschlauch 4. An der Anschlussplatte 10 ist eine Öffnung 11 (vgl. Fig. 2a) der Wundgröße entsprechend ausgeschnitten, so dass nach dem Aufkleben auf die Haut des Patienten ein gasdichter Wundraum 5 im Wundbereich (vgl. Fig. 2b) entsteht. Mit Bezugszahl 25 ist ein umlaufender Rand des Wundabdeckungselementes 3 bezeichnet, der mit der Anschlussplatte 10 fest verklebt ist.

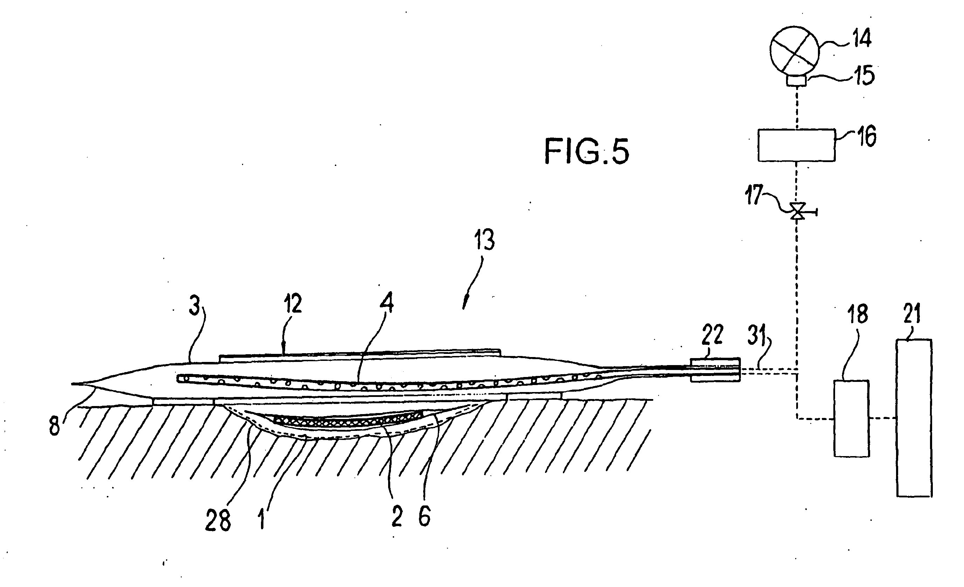

Der abgedichtete Wundraum 5 ist also durch die Wundoberfläche, das Wundabdeckungselement 3 und Innenkante der Öffnung 11 an der Anschlussplatte begrenzt. Dieser Wundraum hat ein variables Volumen, da das Wundabdeckungselement 3 nicht angespannt ist. Zu dem Wundraum, falls er als Unterdruckraum betrachtet wird, zählt auch das Innere des Drainageschlauchs 4 bis zu einem Verschlusselement, wie in der Fig. 5 schematisch gezeigte Rückschlagventil 17.

Der Drainageschlauch 4 ist über ein am Rande des Wundabdeckungselementes 3 angeordnetes Hülsenstück 22 gasdicht unter das Wundabdeckungselement hineingeschoben und ruht auf dem Absorptionskörper 2, ohne mit der Wundoberfläche zu kontaktieren.

Ferner weist die Anschlussplatte 10 an ihren beiden Flachseiten jeweils eine Schutzfolie 19, 24 auf, von denen die dem späteren Wundbereich zugewandte Schutzfolie 19 abziehbar ist. Die andere Schutzfolie 24 ist auf ihrer dem Wundabdeckungselement 3 zugewandten Seite antiadhäsiv ausgerüstet.

Bei dem Absorptionskörper 2 handelt sich um eine Lage 7 (vgl. Fig. 2a) eines mit Superabsorbentien angereicherten Textilabschnittes 33, die mit der flüssigkeitsdurchlässigen Hülle 6 umgeben ist. Die Hülle 6 weist einen an ihrem Umfang liegenden, umlaufenden Überstand 9 an Material auf, wobei die Lage 7 flächenmäßig etwa 40% kleiner als die Hülle 11 ist.

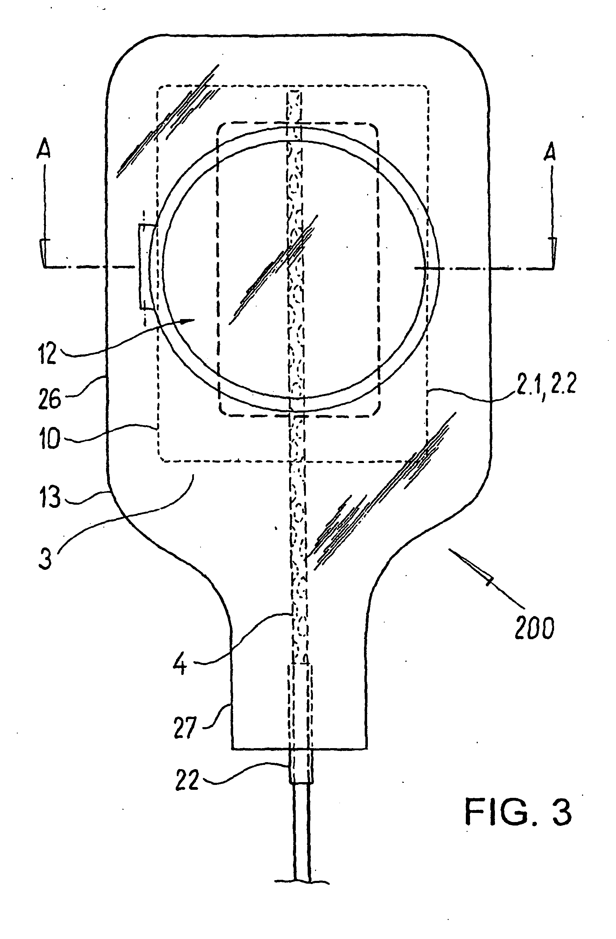

In den Figuren 3 und 5 ist eine der oben beschriebenen ähnliche Drainagevorrichtung 200 dargestellt, bei der das Wundabdeckungselement 3 Teil eines an sich bekannten Versorgungsbeutels 13 mit Hals 27 ist, dessen dem Wundabdeckungselement 3 gegenüber liegende Seitenwand 8 (vgl. Fig. 5) mit der hydrokolloidartigen Anschlussplatte 10 fest verklebt ist, wobei ein Umriss 26 des flach gelegten Versorgungsbeutels über den der Anschlussplatte hinausragt. Im Inneren des auf die Haut des Patienten aufgeklebten Versorgungsbeutels 13 ist der flache Absorptionskörper 2 untergebracht. Mit Bezugszahl 28 ist mit Strichlinie eine Wundkontur 28 bezeichnet.

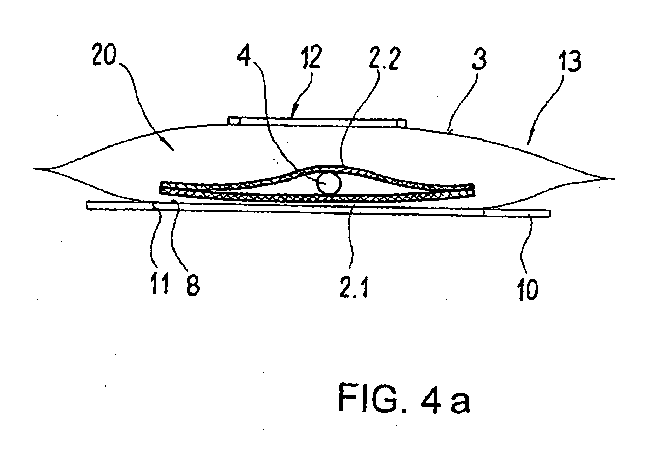

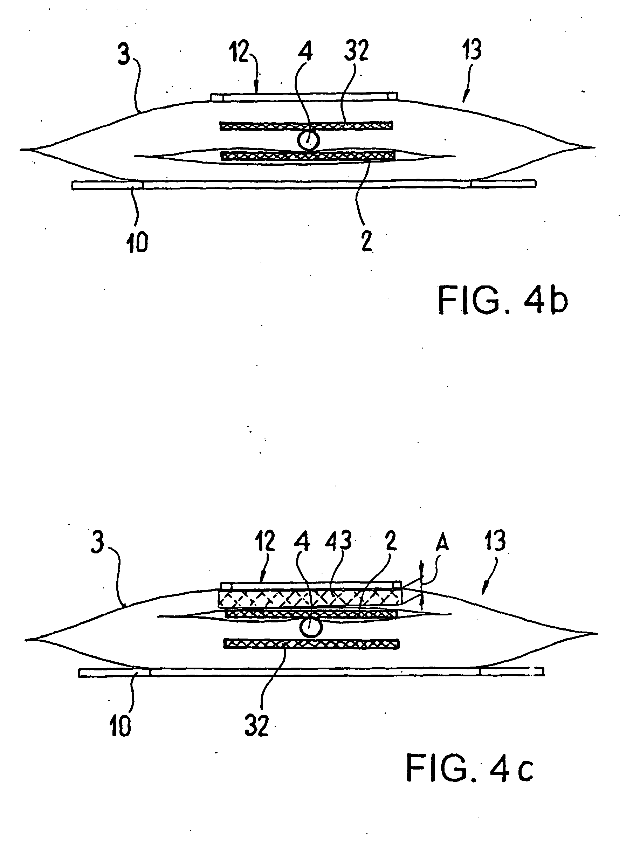

Die Fig. 4a zeigt die Drainagevorrichtung 200 im Schnitt A-A gemäß der Fig.3. Die Besonderheit dieser Ausführungsform ist eine Sandwichanordnung 20 zweier Absorptionskörper 2.1, 2.2, zwischen denen der Drainageschlauch 4 liegt. Die Fig.4b zeigt ebenfalls eine Sandwichanordnung, bei der ein zusätzlicher, nicht mit Superabsorbentien versehener Absorptionskörper 32 zwischen dem Wundabdeckungselement 3 und dem mit Superabsorbentien angereicherten Absorptionskörper 2 liegt. In der Fig. 4c ist wiederum eine Sandwichanordnung dargestellt, bei der der zusätzliche Absorptionskörper 32 zwischen der Anschlussplatte 10 und dem mit Superabsorbentien angereicherten Absorptionskörper 2 liegt. In beiden letzten Fällen ist der Drainageschlauch 4 zwischen den sich voneinander unterscheidenden Absorptionskörpern 2; 32 angeordnet. Der zusätzliche, superabsorberfreie Absorptionskörper 32 liegt in Form eines offenporigen Schaumstoffkörpers vor.

Am Wundabdeckungselement 3 ist eine relativ breite, runde Behandlungsöffnung 12 eingearbeitet, durch die der Absorptionskörper 2 bzw. der zusätzliche Absorptionskörper 32 in den Wundraum 5 einlegbar und aus dem Wundraum entnehmbar ist. Die Behandlungsöffnung 12 ist mit einem Schwenkdeckel 23 gasdicht verschließbar.

In Fig. 1 ist skizzenhaft ein in der Mitte des Schwenkdeckels 23 angeordneter Luftdurchlass 34 gezeigt, mit dem sich der Unterdruck regulieren bzw. derart justieren lässt, dass während des Saugvorgangs innerhalb des Wundraums zu einem Luftdurchzug kommen kann.

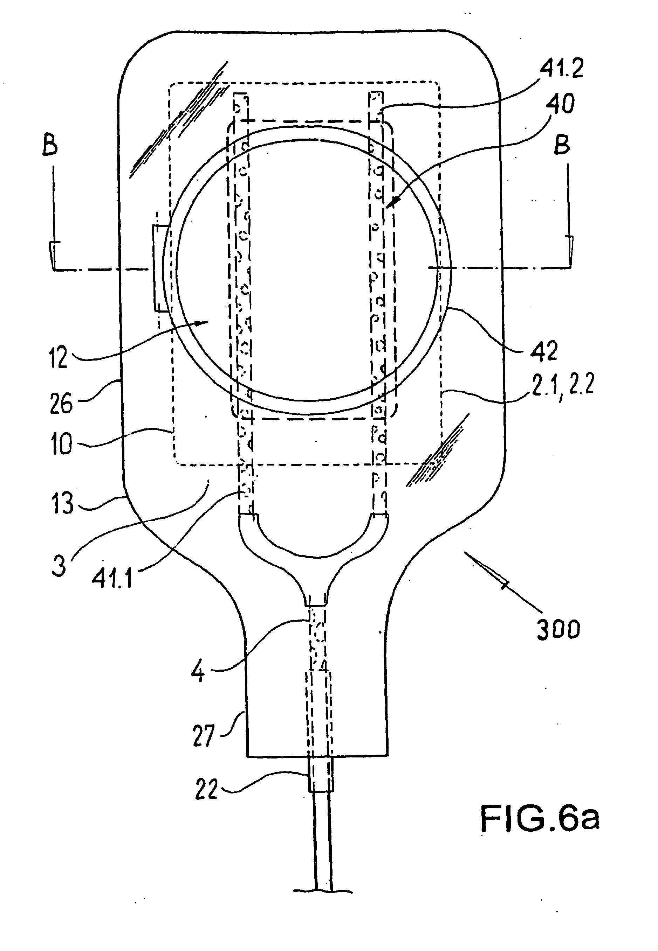

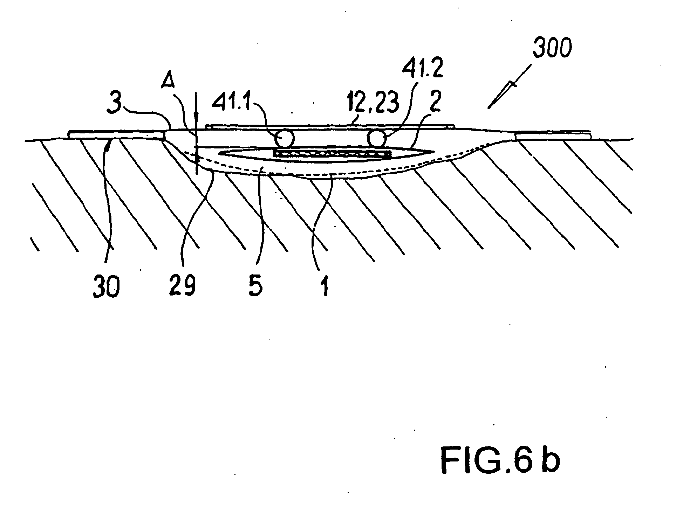

Die Figuren 6a und 6b zeigen eine Drainagevorrichtung 300, die im Wesentlichen der in Fig. 1 dargestellten ähnlich ist. Der Unterschied besteht darin, dass das unterhalb des Wundabdeckungselementes 3 liegende, perforierte Teil des Drainageschlauches 4 gabelförmig ist. Auf dieser Weise ist ein stabiler Druckverteiler 40 entstanden, da die beiden Zweige 41.1, 41.2 auf dem Absorptionskörper 2 aufliegen und während des Saugvorgangs gegen einen versteiften Kranz 42 des Schwenkdeckels 23 drücken.

Ein anderer Druckverteiler 43 liegt in Form eines flachen, offenzelligen Schaumstoffstücks 44 (vgl. Fig. 4c) vor. Das Schaumstoffstück 44 ist kaum komprimierbar, daher behält seine Dicke, die einen erforderlichen Abstand A zwischen dem Wundabdeckungselement 3 und dem Absorptionskörper 2 definiert.

Außerdem ist für die Vorrichtung eine sterile, luftdichte Verpackung (nicht dargestellt) vorgesehen.

### Funktion (vgl. Figuren 2a bis 2d und 5):

Eine tiefe Wunde 29 wird durch das Ankleben der Drainagevorrichtung 100 gemäß der Fig. 1 auf die Haut des Patienten vollständig abgedeckt. Vorher wurde an der Anschlussplatte 10 eine Öffnung 11 ausgeschnitten und danach die in der Fig. 2a gezeigte Schutzfolie 19 entfernt, so dass eine Klebefläche 30 an der Unterseite der Anschlussplatte 10 freigibt.

Mit einer sterilisierten Pinzette (nicht dargestellt) wurde zuerst durch die Behandlungsöffnung 12 das schleimhautfreundliche, perforierte, folienartige Schutzelement 1, dann der flache Absorptionskörper 2 samt Hülle 6 vorsichtig auf die Wundoberfläche gelegt. Der Absorptionskörper 2 weist ein Anfangsvolumen V1 auf. Über das Hülsenstück 22 wird dann der Drainageschlauch 4 unter das Wundabdeckungselement 3 so hineingeschoben, dass er auf der Hülle des Absorptionskörpers 2 ruht (vgl. Fig. 2b). Durch das Ankleben der Drainagevorrichtung auf die Haut ist ein gasdichter Wundraum 5 zwischen dem Wundabdeckungselementes 3 und der Wundoberfläche entstanden.

An den Drainageschlauch 4 wurde über eine Leitung 31 eine handbetätigbare Vakuumpumpe 15, die mit einem Druckmanometer 15 ausgestattet ist (vgl. Fig. 5), angeschlossen. Da der Wundraum 5 abgedichtet ist, können mit Hilfe der Vakuumpumpe 15 die im Wundraum 5 befindlichen Gase evakuiert werden. Den Zustand zeigen die Figuren 2b und 5. Mit Hilfe der Vakuumpumpe 15 wurde innerhalb des Wundraumes 5 ein Unterdruck von etwa 100 ml Hg erzeugt. An der Leitung 31 ist zwischen dem Hülsenstück 22 und der Vakuumpumpe 14 ein Rückschlagventil 17 angeordnet, das den Durchfluss von Gasen und eventuell von flüssigen Wundsekreten nur in Richtung eines der Vakuumpumpe 16 vorgeschalteten Auffangbehälters 16 erlaubt.

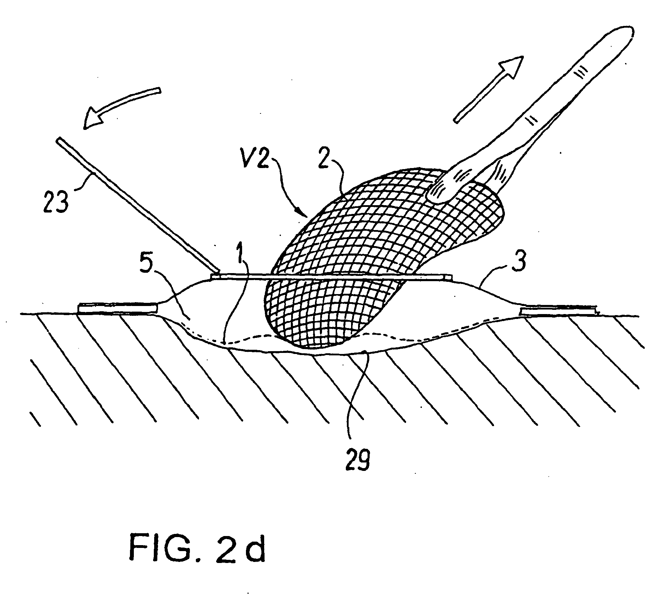

Die aus der Wunde heraustretenden Wundsekrete gelangen in den Absorptionskörper 2 und bewirken eine Kompression unterhalb des Wundabdeckungselementes 3. Nach dem Ansaugen von Wundsekreten nimmt der Absorptionskörpers 2 sein Endvolumen V2 an (vgl. Figuren 2c und 2d). Alle momentan aus der Wunde ausgetretenen Wundsekrete sind von dem Absorptionskörper 2 aufgenommen. Die Außenseite der Hülle 6 bleibt grundsätzlich trocken.

Der verbrauchte Absorptionskörpers 2 und das Schutzelement 1 werden jetzt durch das Anheben des Schwenkdeckels 23 mit Hilfe der Pinzette vorsichtig aus dem Bereich der Wunde entfernt (vgl. Fig. 2d). Nach Bedarf kann auf die Wunde ein neuer Absorptionskörper und ein neues Schutzelement 1 gelegt werden.

Bei sehr stark sezernierenden Wunden kann zum Überschuss an Wundsekrete kommen, die über den Drainageschlauch 4 dem Auffangbehälter 16 zugeleitet werden.

Soll der Patient einer stationären Wundbehandlung unterzogen werden, kann der Unterdruck im Wundraum 5 im Wundbereich durch eine vorhandene klinische Vakuuminstallation 21 (Fig. 5) erzeugt werden. Hierzu empfiehlt sich, an der Leitung zwischen dem Drainageschlauch 4 und der Vakuuminstallation 21 einen Druckregler 18 einzubauen, mit dem sich der Unterdruckbereich einstellen lässt.

### Bezugszeichenliste:

- 1: Schutzelement
- 2: Absorptionskörper
- 3: Wundabdeckungselement
- 4: Drainageschlauch
- 5: Wundraum
- 6: Hülle
- 7: Lage
- 8: Seitenwand
- 9: Überstand
- 10: Anschlussplatte
- 11: Öffnung (v. 10)
- 12: Behandlungsöffnung (v. 3)
- 13: Versorgungsbeutel
- 14: Vakuumpumpe
- 15: Druckmanometer
- 16: Auffangbehälter
- 17: Rückschlagventil
- 18: Druckregler
- 19: Schutzfolie
- 20: Sandwichanordnung
- 21: Vakuuminstallation (stationär)
- 22: Hülsenstück
- 23: Schwenkdeckel
- 24: Schutzfolie
- 25: Rand (v. 3)
- 26: Umriss (v. 13)
- 27: Hals (v. 13)
- 28: Wundkontur
- 29: Wunde
- 30: Klebefläche
- 31: Leitung
- 32: zusätzlicher Absorptionskörper
- 33: Textilabschnitt
- 34: Luftdurchlass

- 40: Druckverteiler
- 41.1: Zweig
- 41.2: Zweig
- 42: Kranz
- 43: Druckverteiler

- 100: Drainagevorrichtung
- 200: Drainagevorrichtung
- 300: Drainagevorrichtung

- A: Abstand

## Patentansprüche

1. Drainagevorrichtung (100; 200; 300) zur Wundbehandlung unter Einsatz von Unterdruck, aufweisend:
- ein gasdichtes, aus folienartigem Material bestehendes Wundabdeckungselement (3), das im am körper des Patienten angelegten Zustand an der Hautoberfläche um den Wundbereich herum adhäsiv befestigt ist und einen zwischen der jeweiligen Wunde und dem Wundabdeckungselement (3) verbleibenden, abgedichteten Wundraum (5) bildet,
- wenigstens einen an ein den Unterdruck erzeugendes Mittel, wie Vakuumpumpe (14), anschließbaren Drainageschlauch (4), der in den Wundraum (5) etwa parallel zum Wundabdeckungselement (3) liegend einsetzbar ist, über den die im Wundraum (5) befindlichen Stoffe evakuiert werden können,
- und wenigstens einen innerhalb des Wundraumes (5) angeordneten, die Wundsekrete aufsaugenden Absorptionskörper (2),
**dadurch gekennzeichnet, dass**
- der Absorptionskörper (2) wenigstens eine in eine Hülle (6) eingefasste Lage (7) eines mit superabsorbierenden Partikeln durchsetzten Textilabschnittes (33) ist,
- wobei besagter Textilabschnitt ein mit Superabsorbern durchsetztes Vlies ist, und
- wobei die Hülle (6) flüssigkeitsdurchlässig ist und Poren aufweist, deren Größe die der superabsorbierenden Partikeln im Wesentlichen nicht überschreitet,
- der in den Wundraum (5) einzulegende Absorptionskörper (2) ein Anfangsvolumen (V1) aufweist, das sich im Laufe des Absorptionsprozesses vergrößert und ein Endvolumen (V2) annimmt, so dass die absorbierten Wundsekrete - bedingt durch die Porengröße der Hülle (11) - innerhalb des Absorptionskörpers (2) und damit unterhalb des Wundabdeckungselementes (3) bis zur Entfernung des Absorptionskörpers aus dem Wundraum verbleiben.

2. Drainagevorrichtung nach Anspruch 1, **dadurch gekennzeichnet, dass** die Lage (7) in Draufsicht auf ihre Flachseite eine Fläche hat, die 3% bis 90% kleiner als die der Hülle (6) ist, damit sich der Absorptionskörper in der Nähe seiner gesamten Füllungskapazität im Querschnitt ungehindert einer Kreisform annähern kann.

3. Drainagevorrichtung nach Anspruch 1, **dadurch gekennzeichnet, dass** das Wundabdeckungselement (3) eine fensterartige Behandlungsöffnung (12) aufweist, durch die der Absorptionskörper (2) in den Wundraum (5) einlegbar und aus dem Wundraum entnehmbar ist, wobei die Behandlungsöffnung (12) mit einem Deckel (23) gasdicht verschließbar ist.

4. Drainagevorrichtung nach einem der Ansprüche 1 bis 3, **dadurch gekennzeichnet, dass** an dem Wundabdeckungselement (3) bzw. am Deckel (23) wenigstens ein Luftdurchlass (34) angeordnet ist, mit dem sich der Unterdruck innerhalb des Wundraums regulieren lässt.

5. Drainagevorrichtung nach Anspruch 1, **dadurch gekennzeichnet, dass** der Textilabschnitt des Absorptionskörpers aus Zellstoff-Vlies besteht.

6. Drainagevorrichtung nach Anspruch 1, **dadurch gekennzeichnet, dass** diese ein flüssigkeitsdurchlässiges, schleimhautfreundliches Schutzelement (1) beinhaltet, das auf einer dem Wundabdeckungselement (3) gegenüber liegenden Seite des Absorptionskörpers (2) angeordnet ist und das flächenmäßig etwa dem umhüllten Absorptionskörper gleich ist.

7. Vorrichtung nach Anspruch 6, **dadurch gekennzeichnet, dass** das Schutzelement (1) folienartig ist.

8. Vorrichtung nach Anspruch 6, **dadurch gekennzeichnet, dass** das Schutzelement (1) ein textiler Materialabschnitt ist.

9. Vorrichtung nach Anspruch 6, **dadurch gekennzeichnet, dass** das Schutzelement (1) aus Schaumstoff besteht.

10. Vorrichtung nach Anspruch 6, **dadurch gekennzeichnet, dass** das Schutzelement (1) eine unterhalb des Absorptionskörpers (2) liegende, lose Schüttung aus Vlies- oder Schaumstoffstücken ist.

11. Drainagevorrichtung nach Anspruch 1, **dadurch gekennzeichnet, dass** das Wundabdeckungselement (3) an wenigstens einem Teil seiner Fläche transparent ist.

12. Drainagevorrichtung nach einem der Ansprüche 1 bis 11, **dadurch gekennzeichnet, dass** das Wundabdeckungselement (3) über eine biegsame Anschlussplatte (10) am Körper des Patienten befestigbar ist, an der wenigstens eine Öffnung (11) eingearbeitet ist.

13. Drainagevorrichtung nach einem der Ansprüche 1 bis 12, **dadurch gekennzeichnet, dass** das Wundabdeckungselement (3) Teil eines Versorgungsbeutels (13) ist, dessen dem Wundabdeckungselement gegenüber liegende Seitenwand (8) mit der Anschlussplatte (10) wenigstens teilweise verklebt ist.

14. Drainagevorrichtung nach einem der Ansprüche 1 bis 13, **dadurch gekennzeichnet, dass** die Hülle (6) des Absorptionskörpers (2) durch Ultraschallnähte vernäht ist.

15. Drainagevorrichtung nach einem der vorhergehenden Ansprüche, **dadurch gekennzeichnet, dass** der Absorptionskörper an seinem Umfang einen Überstand (9) an Hüllenmaterial aufweist.

16. Drainagevorrichtung nach einem der vorhergehenden Ansprüche, **dadurch gekennzeichnet, dass** der Drainageschlauch (4) einem Auffangbehälter (16) zugeleitet ist, welcher Auffangbehälter der Vakuumpumpe (14) bzw. einem Druckmanometer (15) vorgeschaltet ist.

17. Drainagevorrichtung nach einem der vorhergehenden Ansprüche, **dadurch gekennzeichnet, dass** die Vakuumpumpe (14) elektrisch oder mechanisch angetrieben ist.

18. Drainagevorrichtung nach einem der vorhergehenden Ansprüche, **dadurch gekennzeichnet, dass** der Drainageschlauch (4) zwischen zwei eine Sandwichanordnung (20) bildenden Absorptionskörpern (2.1, 2.2; 32) liegt, die gleiche oder unterschiedliche Saugkraft aufweisen.

19. Drainagevorrichtung nach Anspruch 18, **dadurch gekennzeichnet, dass** die Sandwichanordnung (20) einen mit Superabsorbentien durchsetzten Absorptionskörper (2) und wenigstens einen zusätzlichen, nicht mit Superabsorbentien versehenen Absorptionskörper (32) aufweist.

20. Drainagevorrichtung nach Anspruch 14, **dadurch gekennzeichnet, dass** der zusätzliche Absorptionskörper (32) ein flacher, offenporiger Schaumstoffkörper ist.

21. Drainagevorrichtung nach Ansprüchen 19 und 20, **dadurch gekennzeichnet, dass** der zusätzliche Absorptionskörper (32) zwischen der Anschlussplatte (10) und dem mit Superabsorbentien durchsetzten Absorptionskörper (2) liegt.

22. Drainagevorrichtung nach Ansprüchen 19 und 20, **dadurch gekennzeichnet, dass** der zusätzliche Absorptionskörper (32) zwischen dem Wundabdeckungselement (3) und dem mit Superabsorbentien durchsetzten Absorptionskörper (2) liegt.

23. Drainagevorrichtung nach einem der vorhergehenden Ansprüche, **dadurch gekennzeichnet, dass** der Absorptionskörper (2); der zusätzliche schaumstoffartige Absorptionskörper (32) und/oder das Schutzelement (1) silber-, kupfer- oder zinkhaltige Substanzen aufweist, beispielsweise in nanokristalliner Form.

24. Drainagevorrichtung nach einem der vorhergehenden Ansprüche, **dadurch gekennzeichnet, dass** der Absorptionskörper (2); der zusätzliche schaumstoffartige Absorptionskörper (32) und/oder das Schutzelement (1) mit Aktivkohle angereichert ist.

25. Vorrichtung nach einem der vorhergehenden Ansprüche, **dadurch gekennzeichnet, dass** unterhalb des Wundabdeckungselementes (3), jedoch oberhalb des umhüllten Absorptionskörpers (2) oder des zusätzlichen schaumstoffartigen Absorptionskörpers (32) ein mit der Hülle (11) des Absorptionskörpers (2) kontaktierender Druckverteiler (40) angeordnet ist.

26. Vorrichtung nach Anspruch 25, **dadurch gekennzeichnet, dass** der Druckverteiler (40) ein Formstück aus gasdurchlässigem Schaumstoff ist.

27. Vorrichtung nach Anspruch 25, **dadurch gekennzeichnet, dass** der Druckverteiler (40) durch den unterhalb des Wundabdeckungselementes (3) gabel-, schlaufen- oder mäanderartig verlaufenden Drainageschlauch (4) gebildet ist.

28. Drainagevorrichtung nach einem der vorhergehenden Ansprüche, **dadurch gekennzeichnet, dass** diese über einen Druckregler (18) an eine vorhandene stationäre klinische Vakuuminstallation (21) anschließbar ist.

## Claims

1. Drainage device (100; 200; 300) for wound healing using negative pressure, comprising:
- a gas-tight wound covering element (3) consisting of film-like material, which element, having been placed on the body of the patient, is adhesively attached to the skin surface around the region of the wound and forms a sealed wound chamber (5) which remains between the wound in question and the wound covering element (3),
- at least one drainage tube (4) which can be attached to a means for producing the negative pressure such as a vacuum pump (14), can be inserted into the wound chamber (5) lying approximately in parallel with the wound covering element (3), and by means of which the substances in the wound chamber (5) can be evacuated,
- and at least one absorption body (2) disposed within the wound chamber (5) and soaking up the wound secretions,
**characterised in that**
- the absorption body (2) is at least one layer (7), enclosed in a sheath (6), of a textile portion (33) interspersed with superabsorbent particles,
- wherein said textile portion is a vleece interspersed with superabsorbing material
- wherein the sheath (6) is liquid-permeable and has pores, the size of which does not essentially exceed that of the superabsorbent particles,
- the absorption body (2) to be placed into the wound chamber (5) has a starting volume (V1) which is enlarged over the course of the absorption process and assumes a final volume (V2) so that - dependent upon the pore size of the sheath (11) - the absorbed wound secretions remain within the absorption body (2) and therefore below the wound covering element (3) until the absorption body is removed from the wound chamber.

2. Drainage device as claimed in claim 1, **characterised in that** in a plan view onto its flat side the layer (7) has a surface which is 3% to 90% smaller that that of the sheath (6) so that the absorption body can, without hindrance, approach a circular cross-sectional shape when close to its entire filling capacity.

3. Drainage device as claimed in claim 1, **characterised in that** the wound covering element (3) has a window-like operating orifice (12) by means of which the absorption body (2) can be placed into the wound chamber (5) and removed from the wound chamber, wherein the operating orifice (12) can be closed in a gas-tight manner by a cover (23).

4. Drainage device as claimed in any one of claims 1 to 3, **characterised in that** on the wound covering element (3) or on the cover (23) at least one air aperture (34) is disposed, with which the negative pressure within the wound chamber can be regulated.

5. Drainage device as claimed in claim 1, **characterised in that** the textile portion of the absorption body consists of cellulose non-woven fabric.

6. Drainage device as claimed in claim 1, **characterised in that** this device contains a liquid-permeable, mucous membrane-friendly protective element (1) which is disposed on a side of the absorption body (2) opposite the wound covering element (3) and which in terms of its surface is approximately equal to the sheathed absorption body.

7. Device as claimed in claim 6, **characterised in that** the protective element (1) is film-like.

8. Device as claimed in claim 6, **characterised in that** the protective element (1) is a textile material portion.

9. Device as claimed in claim 6, **characterised in that** the protective element (1) consists of foamed material.

10. Device as claimed in claim 6, **characterised in that** the protective element (1) is a loose bulk material of pieces of non-woven fabric or pieces of foamed material which lies underneath the absorption body (2).

11. Drainage device as claimed in claim 1, **characterised in that** the wound covering element (3) is transparent on at least part of its surface.

12. Drainage device as claimed in any one of claims 1 to 11, **characterised in that** the wound covering element (3) can be attached to the body of the patient by means of a flexible attachment plate (10) in which at least one orifice (11) is provided.

13. Drainage device as claimed in any one of claims 1 to 12, **characterised in that** the wound covering element (3) is part of a supply pouch (13), the side wall (8) of which, opposite the wound covering element, is at least partially stuck to the attachment plate (10).

14. Drainage device as claimed in any one of claims 1 to 13, **characterised in that** the sheath (6) of the absorption body (2) is stitched by ultrasonic stitching.

15. Drainage device as claimed in any one of the preceding claims, **characterised in that** on its periphery the absorption body has an overhang (9) of sheath material.

16. Drainage device as claimed in any one of the preceding claims, **characterised in that** the drainage tube (4) is fed to a receiving container (16), which receiving container is connected upstream of the vacuum pump (14) and/or of a pressure gauge (15).

17. Drainage device as claimed in any one of the preceding claims, **characterised in that** the vacuum pump (14) is electrically or mechanically driven.

18. Drainage device as claimed in any one of the preceding claims, **characterised in that** the drainage tube (4) lies between two absorption bodies (2.1, 2.2; 32) which form a sandwich arrangement (20) and have the same or different drawing power.

19. Drainage device as claimed in claim 18, **characterised in that** the sandwich arrangement (20) has an absorption body (2), which is interspersed with superabsorbent materials, and at least one additional absorption body (32) which is not provided with superabsorbent materials.

20. Drainage device as claimed in claim 14, **characterised in that** the additional absorption body (32) is a flat open-pored foamed material body.

21. Drainage device as claimed in claims 19 and 20, **characterised in that** the additional absorption body (32) lies between the attachment plate (10) and the absorption body (2) interspersed with superabsorbent materials.

22. Drainage device as claimed in claims 19 and 20, **characterised in that** the additional absorption body (32) lies between the wound covering element (3) and the absorption body (2) which is interspersed with superabsorbent materials.

23. Drainage device as claimed in any one of the preceding claims, **characterised in that** the absorption body (2); the additional foam-like absorption body (32) and/or the protective element (1) comprises silver-containing, copper-containing or zinc-containing substances, for example in nanocrystalline form.

24. Drainage device as claimed in any one of the preceding claims, **characterised in that** the absorption body (2); the additional foam-like absorption body (32) and/or the protective element (1) is enriched with activated carbon.

25. Device as claimed in any one of the preceding claims, **characterised in that** below the wound covering element (3), but above the sheathed absorption body (2) or the additional foam-like absorption body (32) a pressure distributor (40) contacting the sheath (11) of the absorption body (2) is disposed.

26. Device as claimed in claim 25, **characterised in that** the pressure distributor (40) is a moulded part made from gas-tight foamed material.

27. Device as claimed in claim 25, **characterised in that** the pressure distributor (40) is formed by the drainage tube (4) extending below the wound covering element (3) in a fork-like, loop-like or meandering manner.

28. Device as claimed in any one of the preceding claims, **characterised in that** this device can be attached to a provided stationary clinical vacuum installation (21) by means of a pressure regulator (18).

## Revendications

1. Dispositif de drainage (100; 200; 300) servant à soigner des plaies par application d'une pression négative et comportant:
- un élément de recouvrement de la plaie (3) réalisé en un matériau de type feuille et étanche aux gaz, lequel élément (3), appliqué au corps du patient, est fixé par adhésif à la surface de la peau autour de la plaie en créant, entre ledit élément et la plaie, un espace-plaie (5) étanche,
- au moins un tuyau de drainage (4) apte à être connecté à un dispositif générant la pression négative, tel qu'une pompe à vide (14), lequel tuyau de drainage (4) peut être inséré dans l'espace-plaie (5) pour s'y trouver à plat, à peu près parallèle à l'élément de recouvrement de la plaie (3), et permet d'évacuer les substances se trouvant dans l'espace-plaie (5),
- et au moins un corps d'absorption (2) disposé à l'intérieur de l'espace-plaie (5) et absorbant les sécrétions de plaie,
**caractérisé**
- **en ce que** le corps d'absorption (2) est constitué d'au moins une couche (7) - enveloppée d'une gaine (6) - d'un morceau de textile (33) chargé de particules superabsorbantes,
- le morceau de textile est un non-tissé bourré avec des superabsorbantes,
- la gaine (6) étant perméable aux liquides et présentant des pores dont la taille n'est pas supérieure, pour l'essentiel, à celle des particules superabsorbantes,
- et **en ce que** le corps d'absorption (2) à introduire dans l'espace-plaie (5) présente un volume initial (V1) qui, en augmentant au cours du processus d'absorption, devient un volume final (V2), de sorte que les sécrétions de plaie absorbées restent - du fait de la grosseur des pores de la gaine (6) - à l'intérieur du corps d'absorption (2) et donc en-dessous de l'élément de recouvrement de la plaie (3), et ce jusqu'au retrait du corps d'absorption (2) de l'espace-plaie (5).

2. Dispositif de drainage selon la revendication 1, **caractérisé en ce que** la couche (7), vue en plan par son côté plat, présente une superficie de 3% à 90% inférieure à celle de la gaine (6), afin que le corps d'absorption (2) puisse, à l'approche de sa capacité totale de remplissage, prendre librement une forme qui, vue en coupe, est à peu près circulaire.

3. Dispositif de drainage selon la revendication 1, **caractérisé en ce que** l'élément de recouvrement de la plaie (3) comporte un orifice de soins (12) à travers lequel le corps d'absorption (2) peut être introduit dans l'espace-plaie (5) et en être retiré, l'orifice de soins (12) fermant hermétiquement par le biais d'un couvercle (23) en assurant l'étanchéité aux gaz.

4. Dispositif de drainage selon l'une quelconque des revendications 1 à 3, **caractérisé en ce qu'**il est agencé, sur l'élément de recouvrement de la plaie (3), voire sur le couvercle (23), au moins une prise d'air (34) permettant de régler la pression négative à l'intérieur de l'espace-plaie (5).

5. Dispositif de drainage selon la revendication 1, **caractérisé en ce que** le morceau de textile (33) du corps d'absorption (2) est un non-tissé de cellulose.

6. Dispositif de drainage selon la revendication 1, **caractérisé en ce que** ledit dispositif comporte un élément de protection (1) perméable aux liquides et n'agressant pas la muqueuse, lequel élément de protection (1) est disposé sur un côté du corps d'absorption (2) opposé à l'élément de recouvrement de la plaie (3) et sa superficie est à peu près identique à celle du corps d'absorption (2) gainé.

7. Dispositif selon la revendication 6, **caractérisé en ce que** l'élément de protection (1) est du type feuille.

8. Dispositif selon la revendication 6, **caractérisé en ce que** l'élément de protection (1) est un morceau de textile.

9. Dispositif selon la revendication 6, **caractérisé en ce que** l'élément de protection (1) est en mousse synthétique.

10. Dispositif selon la revendication 6, **caractérisé en ce que** l'élément de protection (1) est un ensemble de fragments de non-tissé ou de mousse synthétique disposés en vrac en-dessous du corps d'absorption (2).

11. Dispositif de drainage selon la revendication 1, **caractérisé en ce que** l'élément de recouvrement de la plaie (3) est transparent sur au moins une portion de sa superficie.

12. Dispositif de drainage selon l'une quelconque des revendications 1 à 11, **caractérisé en ce que** l'élément de recouvrement de la plaie (3) peut être fixé au corps du patient par le biais d'une plaque de jonction (10) souple comportant au moins une ouverture (11).

13. Dispositif de drainage selon l'une quelconque des revendications 1 à 12, **caractérisé en ce que** l'élément de recouvrement de la plaie (3) fait partie d'une poche de soins (13) dont la paroi latérale (8) opposée à l'élément de recouvrement de la plaie (3) est collée, au moins pour partie, à la plaque de jonction (10).

14. Dispositif de drainage selon l'une quelconque des revendications 1 à 13, **caractérisé en ce que** la gaine (6) du corps d'absorption (2) est soudée par ultrasons.

15. Dispositif de drainage selon l'une quelconque des revendications précédentes, **caractérisé en ce que** le corps d'absorption (2) comporte, dépassant de sa circonférence, un supplément de matériau de gainage (9).

16. Dispositif de drainage selon l'une quelconque des revendications précédentes, **caractérisé en ce que** le tuyau de drainage (4) conduit vers un récipient collecteur (16) branché en amont de la pompe à vide (14) ou d'un manomètre (15).

17. Dispositif de drainage selon l'une quelconque des revendications précédentes, **caractérisé en ce que** la pompe à vide (14) est à commande électrique ou mécanique.

18. Dispositif de drainage selon l'une quelconque des revendications précédentes, **caractérisé en ce que** le tuyau de drainage (4) est placé entre deux corps d'absorption (2.1, 2.2; 32) disposés en sandwich et dont la capacité d'absorption est soit identique soit différente.

19. Dispositif de drainage selon la revendication 18, **caractérisé en ce que** l'agencement en sandwich (20) comporte un corps d'absorption (2) chargé de superabsorbants et au moins un corps d'absorption supplémentaire (32) non pourvu de superabsorbants.

20. Dispositif de drainage selon la revendication 19, **caractérisé en ce que** le corps d'absorption supplémentaire (32) est un corps plat en mousse synthétique à pores ouvertes.

21. Dispositif de drainage selon les revendications 19 et 20, **caractérisé en ce que** le corps d'absorption supplémentaire (32) est disposé entre la plaque de jonction (10) et le corps d'absorption (2) chargé de superabsorbants.

22. Dispositif de drainage selon les revendications 19 et 20, **caractérisé en ce que** le corps d'absorption supplémentaire (32) est disposé entre l'élément de recouvrement de la plaie (3) et le corps d'absorption (2) chargé de superabsorbants.

23. Dispositif de drainage selon l'une quelconque des revendications précédentes, **caractérisé en ce que** le corps d'absorption (2), le corps d'absorption supplémentaire (32) en mousse synthétique et/ou l'élément de protection (1) comportent des substances argentifères, cuprifères ou zincifères, par exemple sous forme nanocristalline.

24. Dispositif de drainage selon l'une quelconque des revendications précédentes, **caractérisé en ce que** le corps d'absorption (2), le corps d'absorption supplémentaire (32) en mousse synthétique et/ou l'élément de protection (1) sont enrichis en charbon actif.

25. Dispositif de drainage selon l'une quelconque des revendications précédentes, **caractérisé en ce qu'**il est agencé, en-dessous de l'élément de recouvrement de la plaie (3) mais au-dessus du corps d'absorption (2) gainé ou du corps d'absorption supplémentaire (32) en mousse synthétique, un répartiteur de pression (40) qui est en contact avec la gaine (6) du corps d'absorption (2).

26. Dispositif selon la revendication 25, **caractérisé en ce que** le répartiteur de pression (40) est une pièce moulée en mousse synthétique perméable aux gaz.

27. Dispositif selon la revendication 25, **caractérisé en ce que** le répartiteur de pression (40) est constitué du tuyau de drainage (4) disposé en fourche, en boucle ou en méandres en-dessous de l'élément de recouvrement de la plaie (3).

28. Dispositif de drainage selon l'une quelconque des revendications précédentes, **caractérisé en ce que** ledit dispositif peut être raccordé, par le biais d'un régulateur de pression, à une installation clinique stationnaire de production de vide (21).
